# EUROPÄISCHE PATENTSCHRIFT

(11) **EP 2 288 330 B1**
(45) Veröffentlichungstag und Bekanntmachung des Hinweises auf die Patenterteilung: **27.06.2012**
(21) Anmeldenummer: 08867902.2
(22) Anmeldetag: 22.12.2008
(51) Int. Cl.: A61K 8/06, A61K 8/39, A61K 8/49, A61K 8/58, A61K 8/86, A61K 8/26, A61K 8/28, A61Q 15/00, A61K 8/34, A61K 8/37, A61K 8/894

(54) **TRANSPARENTE SCHWEISSHEMMENDE GELE**
TRANSPARENT ANTIPERSPIRANT GELS
GELS ANTI-TRANSPIRANTS TRANSPARENTS

(30) Priorität: 28.12.2007 DE 102007063351
(43) Veröffentlichungstag der Anmeldung: 02.03.2011
(73) Patentinhaber: Henkel AG & Co. KGaA, 40589 Düsseldorf (DE)
(72) Erfinder: BANOWSKI, Bernhard, 40597 Düsseldorf (DE); CLAAS, Marcus, 40723 Hilden (DE); BUSE, Nadine, 40724 Hilden (DE)
(86) Internationale Anmeldenummer: PCT/EP2008/068194
(87) Internationale Veröffentlichungsnummer: WO 2009/083545

(56) Entgegenhaltungen:
- DE-U1- 29 724 204
- US-A- 6 007 799
- carechemicals: "Cetiol CC product data sheet", , 27. Dezember 2007 (2007-12-27), XP002615041, Gefunden im Internet: URL:http://www.cospha.ro/dbimg/Cetiol%20CC .pdf [gefunden am 2010-12-21]

## Beschreibung

Gegenstand der vorliegenden Anmeldung sind transparente schweißhemmende Zusammensetzungen in Form einer Wasser-in-Öl-Emulsion, die zur Verbesserung der schweißhemmenden Wirkung eine ausgewogene Mischung aus ausgewählten Ölkomponenten und Emulgatoren enthalten.

### Stand der Technik

Schweißhemmende Zusammensetzungen in Form einer Wasser-in-Öl-Emulsion sind im Stand der Technik bereits bekannt. WO 92/05767 A1 offenbart transparente schweißhemmende Gele in Form einer Wasser-in-Öl-Emulsion auf der Basis von Cyclomethiconen und emulgierenden Ethylenoxid-Propylenoxid-substituierten Polydimethylsiloxanen. WO 96/06594 A1 offenbart transparente schweißhemmende Wasser-in-Öl-Zusammensetzungen, die flüchtige Siliconöle und/oder flüchtige Kohlenwasserstofföle und die Silicon-freien Wasser-in-Öl-Emulgatoren Laureth-1 oder Laureth-4 enthalten. WO 98/17238 A1 offenbart schweißhemmende Stifte in Form einer Wasser-in-Öl-Emulsion auf der Basis von mit Alkylresten endständig disubstituierten Silicon-freien Wasser-in-Öl-Emulgatoren, ohne das Problem der Transparenz zu behandeln. WO 00/067888 A1 offenbart dünnflüssige W/O-Emulsionen mit einer Viskosität von maximal 5000 mPas, mindestens 75 Gew.-% Wasserphase, maximal 20 Gew.-% Lipide, Emulgatoren und weitere lipophile Bestandteile, wobei die Ölphase eine Gesamtpolarität zwischen 20 und 30 mN/m aufweist und frei ist von Siliconen, stabilisiert durch mit Alkylresten endständig disubstituierten Silicon-freien Wasser-in-Öl-Emulgatoren. Auch diese Schrift beschäftigt sich nicht mit dem Problem der Transparenz.

US 6,007,799 offenbart transparente schweißhemmende Zusammensetzungen in Form von Wasser-in-Öl Emulsionen, die einen höheren Gehalt an schweißhemmenden Wirkstoffen enthalten und keinen Einsatz von Cyclomethicone erfordern. Die in US 6,007,799 offenbarten Zusammensetzungen enthalten einen alkoxylierten, alkyl-substituierten Siloxan-Emulgator, mindestens einen "coupling agent" ausgewählt aus Verbindungen wie z.B. PPG-14 Butylether und eine Ölphase enthaltend eine Komponente mit Brechungsindex von 1,4 bis 1,5.

### Aufgabe

Transparente Gele in Form einer Wasser-in-Öl-Emulsion erfreuen sich beim Verbraucher großer Beliebtheit. Die bekannten treibgasfreien W/O-Gele auf der Basis von Cyclomethiconen ergeben bei der Applikation auf die Haut ein frisches und gleichzeitig pflegendes Gefühl. Gleichzeitig zeigen sie direkt nach der Applikation zunächst keine Rückstände. Diese - anfangs - weitgehend rückstandsfreie Applikation, die vom Verbraucher sehr geschätzt wird, wird für ihn auch durch die Transparenz der Zusammensetzung visualisiert.
Für optimale Transparenz muss der Brechungsindex der Ölphase und der Wasserphase innerhalb von etwa 0,001 oder besser und bevorzugt innerhalb von etwa 0,0004 aufeinander abgestimmt sein. Bei der Festlegung eines Bestandteils, beispielsweise der besonders effektiven schweißhemmenden Aluminium-Zirconium-Verbindungen, die einen relativ hohen Brechungsindex der wässrigen Phase mit sich bringen, ist man also in der Wahl der übrigen Bestandteile eingeschränkt.

Ein Problem der bekannten Gele ist ihr hoher Gehalt an Cyclomethicone. Bei den handelsüblichen Cyclomethiconen unterscheidet man vor allem Cyclotetrasiloxan, Cyclopentasiloxan und Cyclohexasiloxan. Cyclotetrasiloxan, dessen Schmelzpunkt mit -11°C ungewöhnlich hoch liegt, kann in den für ein Wasser-in-Öl-Emulsionsgel typischen höheren Einsatzmengen zu Problemen bei der Lagerstabilität führen. Außerdem sieht man heutzutage aus ökologischen Gründen weitgehend von einem Einsatz des Cyclotetrasiloxans ab. Die üblichen Cyclomethicone-Handelsprodukte sind nahezu frei von Cyclotetrasiloxan. Dennoch ist die Substanzklasse der Cyclomethicone aufgrund selbst des Spurengehalts an Cyclotetrasiloxan ein problematischer Rohstoff. Andererseits weisen die Cyclomethicone hervorragende Gebrauchseigenschaften auf, so dass ihr Ersatz ausgesprochen schwierig ist. Cyclomethicone sind relativ flüchtige Ölkomponenten. Aus diesem Grund werden sie gern in Kosmetika, insbesondere in Antitranspirantien, eingesetzt, da sie helfen, das Problem des Anschmutzens der Kleidung zu lösen. Andererseits bilden Antitranspirantien mit einem zu hohen Anteil an flüchtigem Cyclomethicone nach dem Antrocknen, das heißt erst einige Zeit nach der Applikation, auf der Haut weiße Rückstände, die schlecht auf der Haut haften und herunterrieseln können, was von vielen Verbrauchern als unangenehm empfunden wird.

Eine Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen sind.
Eine weitere Aufgabe der vorliegenden Anmeldung war es, transparente schweißhemmende Zusammensetzungen in Form einer Wasser-in-Öl-Emulsion bereitzustellen, die einen hohen Gehalt an dispergierter wässriger Phase, vorzugsweise mindestens 75 Gew.-%, aufweisen. Da die handelsüblichen schweißhemmenden Wirkstoffe wasserlöslich sind, befinden sie sich in der wässrigen Phase. Eine schnelle Freisetzung des Wirkstoffes kann dabei durch einen hohen Gehalt an wässriger Phase unterstützt werden.
Eine weitere Aufgabe der vorliegenden Anmeldung war es, transparente schweißhemmende Zusammensetzungen in Form einer Wasser-in-Öl-Emulsion bereitzustellen, die eine gelartige Konsistenz mit mittlerer bis hoher Viskosität aufweisen und mittels einer Applikationskugel (Roll-on) oder einer stiftähnlichen Hülse aufgetragen werden können.
Eine weitere Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen sind und eine gute Freisetzung des schweißhemmenden Wirkstoffs zeigen.
Eine weitere Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen und ausreichend lagerstabil sind.
Eine weitere Aufgabe der vorliegenden Anmeldung war es, transparente schweißhemmende Zusammensetzungen in Form einer Wasser-in-Öl-Emulsion bereitzustellen, die weitgehend oder sogar vollständig frei von Cyclomethiconen und ausreichend lagerstabil sind und eine gute Freisetzung des schweißhemmenden Wirkstoffs zeigen.

Überraschend wurde festgestellt, dass Zusammensetzungen gemäß Anspruch 1 die gestellten Aufgaben hervorragend lösen.

Gegenstand der vorliegenden Anmeldung sind transparente schweißhemmende Zusammensetzungen in Form einer Wasser-in-Öl-Emulsion, enthaltend 10-20 Gew.-% äußere Ölphase, enthaltend i) mindestens einen symmetrischen, unsymmetrischen oder cyclischen Ester der Kohlensäure mit linearen oder verzweigten C6 - C22-Alkanolen, ii) mindestens ein Anlagerungsprodukt von 1- 14 Propylenoxid-Einheiten an ein- oder mehrwertige C₃₋₁₆Alkanole sowie iii) 0 bis maximal 3 Gew.-% Cyclomethicone, 75 - 90 Gew.-% dispergierte wässrige Phase mit mindestens einem schweißhemmenden Wirkstoff, ausgewählt aus Aluminium-Zirconium-Verbindungen, und mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3-20 Ethylenoxid-Einheiten, mindestens einen Silicon-basierten Wasser-in-Öl-Emulgator mit mindestens einem Alkylsubstituenten R mit 4 - 20 Kohlenstoffatomen, mindestens einen Polyethylenglycolether eines linearen oder verzweigten C12 - C22-Alkanols mit 20-150 Ethylenoxideinheiten und keinen Silicon-freien Wasser-in-Öl-Emulgator. Dabei beziehen sich alle Gew.-%-Angaben auf das Gesamtgewicht der Emulsion.

Ein weiterer Gegenstand der vorliegenden Anmeldung ist ein nicht-therapeutisches, kosmetisches Verfahren zur Reduzierung der Schweißbildung, bei dem eine erfindungsgemäße oder erfindungsgemäß bevorzugte Zusammensetzung gemäß einem der Ansprüche 1-9 auf die Haut, bevorzugt auf die Haut im Achselbereich, aufgetragen wird.

Die erfindungsgemäßen Zusammensetzungen enthalten 10-20 Gew.-% äußere Ölphase; bevorzugt beträgt der Gehalt an Ölphase 12 - 18 Gew.%, besonders bevorzugt 14 - 16 Gew.-%, jeweils bezogen auf das Gesamtgewicht der Emulsion. Der Silicon-basierte Wasser-in-Öl-Emulgator sowie gegebenenfalls weitere emulgierende Bestandteile werden im Sinne dieser Anmeldung nicht zur

Ölphase gezählt. Lipophile Bestandteile, die unter Normalbedingungen nicht flüssig ("Öl"), sondern fest vorliegen, z. B. Wachse, werden im Sinne dieser Anmeldung zur Ölphase gezählt. Parfümöle werden im Sinne dieser Anmeldung zur Ölphase gezählt. Gegebenenfalls enthaltenes Ethanol sowie die mehrwertigen C₂ - C₉-Alkanole mit 2 - 6 Hydroxylgruppen und die wasserlöslichen Polyethylenglycole mit 3-20 Ethylenoxid-Einheiten werden im Sinne dieser Anmeldung nicht zur Ölphase, sondern zur Wasserphase gezählt, auch wenn sie zumindest teilweise öllösliche Eigenschaften aufweisen.

"Normalbedingungen" sind im Sinne der vorliegenden Anmeldung eine Temperatur von 20°C und ein Druck von 1013,25 mbar. Schmelzpunktangaben beziehen sich ebenfalls auf einen Druck von 1013,25 mbar.

Die Ölphase enthält zwingend eine Kombination aus mindestens einem symmetrischen, unsymmetrischen oder cyclischen Ester der Kohlensäure mit linearen oder verzweigten C6 - C22-Alkanolen, und mindestens einem Anlagerungsprodukt von 1-14 Propylenoxid-Einheiten an ein- oder mehrwertige C₃-₁₆-Alkanole.
Überraschend wurde festgestellt, dass diese Kombination aus Ölkomponenten einen hervorragenden Ersatz für den Cyclomethicone-Anteil in bekannten schweißhemmenden W/O-Emulsionen darstellt. Diese Ölkombinationen unterstützen in überraschender Weise die Wirkstofffreisetzung des schweißhemmenden Wirkstoffs. Weiterhin bieten diese Öle ein ähnlich angenehmes Hautgefühl wie Cyclomethicone. Weiterhin lassen sich mit diesen Ölen aufgrund ihrer Brechungsindices sehr gut hochtransparente W/O-Emulsionen herstellen.

Bevorzugte Ölkomponenten, die symmetrische, unsymmetrische oder cyclische Ester der Kohlensäure mit linearen oder verzweigten C6 - C22-Alkanolen darstellen, sind Di-n-hexylcarbonat, Di-n-heptylcarbonat, Di-n-octylcarbonat, das unter der INCl-Bezeichnung Dicaprylyl Carbonate beispielsweise als Handelsprodukt Cetiol^{®} CC von Cognis erhältlich ist, weiterhin Di-2-ethylhexylcarbonat, das unter der INCl-Bezeichnung Diethylhexyl Carbonate beispielsweise als Handelsprodukt Tegosoft^{®} DEC von Degussa-Evonik erhältlich ist, weiterhin Di-n-nonylcarbonat, Di-n-decylcarbonat, Di-n-laurylcarbonat, Di-n-myristylcarbonat, Di-n-cetylcarbonat, Di-isocetylcarbonat, Di-n-stearylcarbonat, Di-isostearylcarbonat, Di-arachylcarbonat und Di-behenylcarbonat als symmetrisch substituierte Carbonate, sowie unsymmetrisch substituierte Carbonate, wie n-Hexyl-n-octylcarbonat, n-Hexyl-2-ethylhexylcarbonat und n-Octyl-n-decylcarbonat. Cyclische Ester der Kohlensäure sind erhältlich durch Umesterung von Dimethylcarbonat oder Diethylcarbonat mit zwei- oder mehrwertigen Alkanolen. Außerordentlich bevorzugt sind Di-n-octylcarbonat, das einen Brechungsindex (bei 21 °C) n_{D} von 1,435 - 1,436 aufweist, sowie Di-2-ethylhexylcarbonat. Sie sind bevorzugt in einer Gesamtmenge von 8-18 Gew.-%, besonders bevorzugt 10-16 Gew.-%, außerordentlich bevorzugt 12-14 Gew.-%, bezogen auf die gesamte Emulsion, enthalten.

Bevorzugte Ölkomponenten, die Anlagerungsprodukte von 1-14 Propylenoxid-Einheiten an ein- oder mehrwertige C₃-₁₆-Alkanole darstellen, sind ausgewählt aus den Anlagerungsprodukten von 1 bis 14 Propylenoxid-Einheiten an ein- oder mehrwertige C₃₋₁₆-Alkanole, insbesondere an n-Butanol-1, n-Pentanol-1, n-Hexanol-1, n-Heptanol-1, n-Octanol-1, n-Decanol-1, n-Decan-1, 10-diol, Laurylalkohol, Myristylalkohol und Cetylalkohol. Besonders bevorzugt sind PPG-2-Myristylether, PPG-3-Myristylether (Witconol^{®} APM), PPG-4-Myristylether, PPG-5-Myristylether, PPG-2-Laurylether, PPG-3-Laurylether, PPG-4-Laurylether, PPG-5-Laurylether, PPG-6-Laurylether, PPG-2-Decylether, PPG-3-Decylether, PPG-4-Decylether, PPG-5-Decylether, PPG-6-Decylether, PPG-7-Decylether, PPG-2-Octylether, PPG-3-Octylether, PPG-4-Octylether, PPG-5-Octylether, PPG-6-Octylether, PPG-7-Octylether, PPG-8-Octylether, PPG-2-Hexylether, PPG-3-Hexylether, PPG-4-Hexylether, PPG-5-Hexylether, PPG-6-Hexylether, PPG-7-Hexylether, PPG-8-Hexylether, PPG-9-Hexylether, PPG-4-Butylether, PPG-5-Butylether, PPG-6-Butylether, PPG-7-Butylether, PPG-8-Butylether, PPG-9-Butylether, PPG-10-Butylether, PPG-11-Butylether, PPG-12-Butylether, PPG-13-Butylether und PPG-14-Butylether (Ucon^{®} Fluid AP).
Es wurde festgestellt, dass mit einem PPG-Anlagerungsprodukt wie PPG-15-Steaylether (Arlamol^{®} E) nicht die gleichen guten Effekte in Bezug auf die Freisetzung des schweißhemmenden Wirkstoffs erzielt werden konnten wie mit den Anlagerungsprodukten von 1-14 Propylenoxid-Einheiten an ein-oder mehrwertige C₃₋₁₆-Alkanole.
Erfindungsgemäß besonders bevorzugt sind PPG-3-Myristylether und PPG-14-Butylether; außerordentlich bevorzugt ist PPG-3-Myristylether.
Erfindungsgemäß außerordentlich bevorzugt sind Kombinationen aus Di-n-octylcarbonat und PPG-3-Myristylether, Di-2-ethylhexylcarbonat und PPG-3-Myristylether, Di-n-octylcarbonat und PPG-14-Butylether, sowie Di-2-ethylhexylcarbonat und PPG-14-Butylether.
Das mindestens eine Anlagerungsprodukt von 1-14 Propylenoxid-Einheiten an ein- oder mehrwertige C₃₋₁₆-Alkanole ist bevorzugt in einer Gesamtmenge von 1-5 Gew.-%, besonders bevorzugt 2-4 Gew.-%, außerordentlich bevorzugt 2,5 - 3 Gew.-%, bezogen auf die gesamte Emulsion, enthalten.

Erfindungsgemäß besonders bevorzugte Zusammensetzungen sind dadurch gekennzeichnet, dass die Ölkomponenten, die symmetrische, unsymmetrische oder cyclische Ester der Kohlensäure mit linearen oder verzweigten C6 - C22-Alkanolen darstellen, in einem Gewichtsverhältnis von 4 - 19 zu den Ölkomponenten, die Anlagerungsprodukte von 1-14 Propylenoxid-Einheiten an ein- oder mehrwertige C₃₋₁₆-Alkanole darstellen, vorliegen. Bevorzugt beträgt dieses Gewichtsverhältnis 5-15, besonders bevorzugt 6-14 und außerordentlich bevorzugt 7 - 13. Es wurde festgestellt, dass mit einem Gewichtsverhältnis unter 2 nicht die gleichen guten Effekte in Bezug auf die Freisetzung des schweißhemmenden Wirkstoffs erzielt werden konnten.

Die erfindungsgemäßen Zusammensetzungen sind dadurch gekennzeichnet, dass 0 bis maximal 3 Gew.%, bevorzugt 0 bis maximal 2,5 Gew.-%, besonders bevorzugt 0 bis maximal 2 Gew.-%, außerordentlich bevorzugt 0 bis maximal 1 Gew.-%, Cyclomethicone enthalten sind, jeweils bezogen auf das Gesamtgewicht der Emulsion.

Die erfindungsgemäßen Zusammensetzungen enthalten weiterhin 75 - 90 Gew.-% dispergierte wässrige Phase mit mindestens einem schweißhemmenden Wirkstoff und mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3-20 Ethylenoxid-Einheiten.

Bevorzugt beträgt der Gehalt an wässriger Phase 78 - 88 Gew.-%, besonders bevorzugt 80 - 86 Gew.-%, und außerordentlich bevorzugt 81-84 Gew.%, jeweils bezogen auf das Gesamtgewicht der Emulsion.

Der Gehalt an freiem Wasser beträgt 0-50 Gew.-%, bevorzugt 5-40 Gew.-%, besonders bevorzugt 10-30 Gew.-% und außerordentlich bevorzugt 15-20 Gew.-%, jeweils bezogen auf das Gesamtgewicht der Emulsion. Gebundenes Kristallwasser, beispielsweise aus den schweißhemmenden Wirkstoffen, zählt hierbei nicht zum freien Wasser.

Emulgierende Bestandteile werden im Sinne dieser Anmeldung nicht zur wässrigen Phase gezählt.

Die wässrige Phase der erfindungsgemäßen Zusammensetzungen umfasst mindestens einen schweißhemmenden Wirkstoff, auch als Antitranspirant-Wirkstoff bezeichnet, ausgewählt aus den wasserlöslichen adstringierenden anorganischen und organischen Aluminium-Zirconium-Verbindungen. Aluminium-Zirconium-Verbindungen zeichnen sich durch eine besonders hohe Effizienz aus bei gleichzeitig guter Hautverträglichkeit. Sie verleihen der wässrigen Phase einen höheren Brechungsindex n_{D} als Zirconium-freie Aluminium-Verbindungen. Besonders bevorzugte Antitranspirant-Wirkstoffe sind ausgewählt aus Aluminiumzirkonium-Glycol-Komplexen, z. B. Aluminiumzirkonium-Propylenglycol-Komplexen, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin und Aluminiumzirconiumoctachlorhydrexglycin. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind. Die Antitranspirant-Wirkstoffe können als wässrige oder glycolische Lösung oder als wässriges oder glycolisches Solubilisat eingesetzt werden.
Besonders bevorzugte erfindungsgemäße Zusammensetzungen sind dadurch gekennzeichnet, dass der mindestens eine Antitranspirant-Wirkstoff in einer Menge von 3-27 Gew.-%, vorzugsweise 5-22 Gew.-% und insbesondere 10 - 20 Gew.%, enthalten ist, bezogen auf das Gesamtgewicht der Aktivsubstanz (USP, US Pharmacopoeia) in der Gesamtzusammensetzung. Die in der vorliegenden Patentanmeldung in Gew.-% angegebene Menge an schweißhemmendem/n Salze/n ist nach der Methode der US Pharmacopoeia (USP) zu berechnen, nach der gebundenes Kristallwasser und andere Liganden, z. B. Glycin, ausgeschlossen sind.
Die Verwendung von Aluminium-Zirkonium-Tetrachlorohydrex-Glycin-Komplexen, die beispielsweise von Reheis unter der Bezeichnung Rezal^{®} 36 G Rezal^{®} 36 G C solution im Handel sind, kann erfindungsgemäß besonders bevorzugt sein.

Die wässrige Phase der erfindungsgemäßen Zusammensetzungen umfasst weiterhin mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3-20 Ethylenoxid-Einheiten. Der Zusatz dieser Verbindungen ist insbesondere nötig, um die Transparenz der gesamten Emulsion zu erreichen. Der Brechungsindex der wässrigen Phase ohne diese Polyole wäre zu niedrig, um an den Brechungsindex der Ölphase angeglichen werden zu können. Außerdem tragen diese wasserlöslichen Polyolkomponenten zur Stabilität der Emulsion bei.
Bevorzugt sind diese wasserlöslichen Polyolkomponenten ausgewählt aus 1,2-Propylenglycol, 1,3-Propylenglycol, 2-Methyl-1,3-propandiol, Glycerin, Butylenglycolen wie bevorzugt 1,2-Butylenglycol, besonders bevorzugt 1,3-Butylenglycol, und 1,4-Butylenglycol, Pentylenglycolen wie 1,2-Pentandiol und 1,5-Pentandiol, Hexandiolen, wie besonders bevorzugt 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, sowie Mischungen der vorgenannten Substanzen. Bevorzugte wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 besonders bevorzugt sind. Auch Zucker, Zuckeralkohole und bestimmte Zuckerderivate wie Erythrit, Sorbit, Xylitol, Fructose, Glucose, Maltose, Maltitol, Mannit, Inosit, Sucrose, Trehalose und Xylose können erfindungsgemäß eingesetzt werden, können jedoch aufgrund ihrer Klebrigkeit weniger geeignet oder nur in geringen Mengen geeignet sein.
Bevorzugt sind 1,2-Propylenglycol, 1,3-Butylenglycol, Dipropylenglycol und 1,2-Hexandiol sowie Mischungen hiervon. Bevorzugte Polyolmischungen enthalten 1,2-Propylenglycol und Dipropylenglycol. Besonders bevorzugte Polyolmischungen enthalten 1,2-Propylenglycol und Dipropylenglycol im Gewichtsverhältnis von 4:1 bis 2:1. Diese Mischungen zeigen in Bezug auf Transparenz, Wirkstofffreisetzung, Hautgefühl und Hautverträglichkeit besonders ausgewogene Eigenschaften. Bevorzugte erfindungsgemäße Zusammensetzungen sind dadurch gekennzeichnet, dass die mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3-20 Ethylenoxid-Einheiten, in einer Gesamtmenge von 20 - 60 Gew.-%, bevorzugt 25 - 55 Gew.-%, besonders bevorzugt 30 - 50 Gew.-%, außerordentlich bevorzugt 35 - 45 Gew.-%, jeweils bezogen auf das Gesamtgewicht der Emulsion, enthalten ist.

Die erfindungsgemäßen Zusammensetzungen umfassen weiterhin mindestens einen Silicon-basierten Wasser-in-Öl-Emulgator mit mindestens einem Alkylsubstituenten R mit 4 - 20 Kohlenstoffatomen.
Eine erfindungsgemäß besonders bevorzugte Gruppe von Wasser-in-Öl-Emulgatoren sind die Poly-(C₂-C₃)alkylenglycol-modifizierten Silicone, die mit C₄-C₂₀-Alkylgruppen hydrophob modifiziert sind. Der hydrophobe Alkylsubstituenten R mit 4 - 20 Kohlenstoffatomen ist bevorzugt ausgewählt aus n-Butyl, n-Pentyl, n-Hexyl, n-Heptyl, n-Octyl, 2-Ethylhexyl, n-Decyl, n-Undecyl, n-Dodecyl, n-Tetradecyl, n-Hexadecyl = Cetyl, n-Octadecyl = Stearyl und n-Eicosanyl = Arachyl. Außerordentlich bevorzugt sind Cetylsubstituenten.

Besonders bevorzugte Silicon-basierte Wasser-in-Öl-Emulgatoren sind ausgewählt aus Cetyl PEG/PPG-10/1 Dimethicone (früher: Cetyl Dimethicone Copolyol, erhältlich als Abil EM 90), weiterhin bevorzugt Cetyl PEG/PPG-7/3 Dimethicone, PEG/PPG-10/3 Oleyl Ether Dimethicone, Lauryl Dimethicone PEG-15 Crosspolymer, Cetyl PEG/PPG-15/15 Butyl Ether Dimethicone, Alkyl Methicone Copolyole und Alkyl Dimethicone Ethoxy Glucoside. Außerordentlich bevorzugt ist Cetyl PEG/PPG-10/1 Dimethicone.

Zusätzlich zu den oben aufgeführten hydrophob Alkyl-modifizierten Silicon-basierten Wasser-in-Öl-Emulgatoren können in einer bevorzugten Ausführungsform der Erfindung solche Silicon-basierten Wasser-in-Öl-Emulgatoren enthalten sein, deren frühere INCl-Bezeichnung Dimethicone Copolyol lautete, mit den aktuellen INCl-Bezeichnungen PEG-x Dimethicone (mit x = 2 - 20, bevorzugt 3 - 17, besonders bevorzugt 11 - 12), PEG/PPG a/b Dimethicone (wobei a und b unabhängig voneinander für Zahlen von 2 - 30, bevorzugt 3-30 und besonders bevorzugt 12-20, insbesondere 14 - 18, stehen), Bis-PEG/PPG-c/d Dimethicone (wobei c und d unabhängig voneinander für Zahlen von 10 - 25, bevorzugt 14-20 und besonders bevorzugt 14 - 16, stehen) und Bis-PEG/PPG-e/f PEG/PPG g/h Dimethicone (wobei e, f, g und h unabhängig voneinander für Zahlen von 10 - 20, bevorzugt 14-18 und besonders bevorzugt 16, stehen). Besonders bevorzugt sind PEG/PPG-18/18 Dimethicone, das in einer 1:9-Mischung mit Cyclomethicone als DC 3225 C bzw. DC 5225 C im Handel erhältlich ist, PEG/PPG-4/12 Dimethicone, das unter der Bezeichnung Abil B 8852 erhältlich ist, sowie Bis-PEG/PPG-14/14 Dimethicone, das in einer Mischung mit Cyclomethicone als Abil EM 97 (Goldschmidt) im Handel erhältlich ist, Bis-PEG/PPG-20/20 Dimethicone, das unter der Bezeichnung Abil B 8832 erhältlich ist, PEG/PPG-5/3 Trisiloxane (Silsoft 305), sowie PEG/PPG-20/23 Dimethicone (Silsoft 430 und Silsoft 440).

Bevorzugte erfindungsgemäße Zusammensetzungen sind dadurch gekennzeichnet, dass der mindestens eine Silicon-basierte Wasser-in-Öl-Emulgator mit mindestens einem Alkylsubstituenten R mit 4 - 20 Kohlenstoffatomen in einer Gesamtmenge von 1-4 Gew.-%, bevorzugt 1,5 - 3,5 Gew.-%, besonders bevorzugt 2-3 Gew.-%, außerordentlich bevorzugt 2,2 - 2,5 Gew.-%, jeweils bezogen auf das Gesamtgewicht der Emulsion, enthalten ist.

Die erfindungsgemäßen Zusammensetzungen sind auch ohne Silicon-freie Wasser-in-Öl-Emulgatoren stabil und daher frei von ihnen. Es wurde sogar festgestellt, dass einige Silicon-freie Wasser-in-Öl-Emulgatoren die Wirkstofffreisetzung der erfindungsgemäßen Zusammensetzungen negativ beeinflussen können.
Beispiele für solche erfindungsgemäß ausgeschlossenen Silicon-freien W/O-Emulgatoren sind ausgewählt aus Substanzen der allgemeinen Formel A-O-(CHR¹-X-CHR²-O-)ₐ-A', wobei A und A' gleiche oder verschiedene hydrophobe organische Reste darstellen, a eine Zahl von 1 bis 100, vorzugsweise 2 bis 60, insbesondere 5 bis 40 darstellt, X eine Einfachbindung oder die Gruppe >CHOR³ darstellt, R¹ und R² ein Wasserstoffatom oder eine Methylgruppe darstellen und so gewählt werden, dass nicht beide Reste gleichzeitig Methyl darstellen, und R³ ein Wasserstoffatom oder eine verzweigte oder unverzweigte, gesättigte oder ungesättigte Alkyl- oder Acylgruppe mit 1 bis 20 Kohlenstoffatomen darstellt.
Weitere ausgeschlossene siliconfreie W/O- Emulgatoren sind so gewählt, dass die Reste A und A' gewählt sind aus der Gruppe der verzweigten und unverzweigten, gesättigten und ungesättigten Alkyl-und Acylreste und Hydroxyacylreste mit 10 bis 30 Kohlenstoffatomen sowie ferner aus der Gruppe der über Esterfunktionen miteinander verbundenen Hydroxyacylgruppen, nach dem Schema: OOC-R"-CR'H-(OOC-R"-CR'H)_{b}-OOC-R"-CHR', wobei R' gewählt wird aus der Gruppe der verzweigten und unverzweigten Alkylgruppen mit 1 bis 20 Kohlenstoffatomen und R" gewählt wird aus der Gruppe der verzweigten und unverzweigten Alkylengruppen mit 1 bis 20 Kohlenstoffatomen und b Zahlen von 0 bis 200 annehmen kann.
Weitere ausgeschlossene siliconfreie W/O-Emulgatoren sind ausgewählt aus
(1) gesättigten Alkoholen mit 8 - 24 C-Atomen, insbesondere mit 16 - 22 C-Atomen, z. B. Cetylalkohol, Stearylalkohol, Arachidylalkohol oder Behenylalkohol oder Gemischen dieser Alkohole, wie sie bei der technischen Hydrierung von pflanzlichen und tierischen Fettsäuren erhalten werden;
(2) ethoxylierten Alkoholen und Carbonsäuren mit 8 - 24 C-Atomen, insbesondere mit 16 - 22 C-Atomen, die einen HLB-Wert von 1 - 8 aufweisen, z. B. Laureth-1 oder Laureth-4;
(3) Partialestern aus einem Polyol mit 3 - 6 C-Atomen und gesättigten und/oder ungesättigten, verzweigten und/oder unverzweigten Fettsäuren mit 8 - 24, insbesondere 12 - 18 C-Atomen. Solche Partialester sind z. B. die Monoglyceride von Palmitin-, Stearinsäure und Ölsäure, die Sorbitanmono- und/oder -diester, insbesondere diejenigen von Myristinsäure, Palmitinsäure, Stearinsäure oder von Mischungen dieser Fettsäuren. Hier sind auch die Monoester aus Trimethylolpropan, Erythrit oder Pentaerythrit und gesättigten Fettsäuren mit 14 - 22 C-Atomen zu nennen. Auch die technischen Monoester, die durch Veresterung von 1 Mol Polyol mit 1 Mol Fettsäure erhalten werden, und ein Gemisch aus Monoester, Diester, Triester und ggf. unverestertem Polyol darstellen, sind einsetzbar.
(4) Polyglycerinestern gesättigter und/oder ungesättigter, verzweigter und/oder unverzweigter Alkancarbonsäuren einer Kettenlänge von 8 - 24, insbesondere 12 - 18 C-Atomen, mit bis zu 10 Glycerineinheiten, vorzugsweise bis zu 3 Glycerineinheiten und einem Veresterungsgrad von 1-10, vorzugsweise 1-5;
(5) Mono- und/oder Polyglycerinethern gesättigter und/oder ungesättigter, verzweigter und/oder unverzweigter Alkohole einer Kettenlänge von 8 - 30, insbesondere 12 - 18 C-Atomen, mit bis zu 10 Glycerineinheiten, vorzugsweise bis zu 3 Glycerineinheiten und einem Veretherungsgrad von 1-10, vorzugsweise 1-5;
(6) Propylenglycolestern gesättigter und/oder ungesättigter, verzweigter und/oder unverzweigter Alkancarbonsäuren einer Kettenlänge von 8 - 24, insbesondere 12 - 18 C-Atomen;
(7) Methylglucose-Estern gesättigter und/oder ungesättigter, verzweigter und/oder unverzweigter Alkancarbonsäuren einer Kettenlänge von 8 - 24, insbesondere 12 - 18 C-Atomen;
(8) Polyglycerin-Methylglucose-Estern gesättigter und/oder ungesättigter, verzweigter und/oder unverzweigter Alkancarbonsäuren einer Kettenlänge von 8 - 24, insbesondere 12 - 18 C-Atomen.

Weitere Beispiele für erfindungsgemäß ausgeschlossene Silicon-freie W/O-Emulgatoren sind Glyceryllanolat, Glycerylmonostearat, Glyceryldistearat, Glycerylmonoisostearat, Glycerylmonomyristat, Glycerylmonooleat, Diglycerylmonostearat, Glycerylmonolaurat, Glycerylmonocaprinat, Glycerylmonocaprylat, Diglycerylmonoisostearat, Diglyceryldiisostearat, Propylenglycolmonostearat, Propylenglycolmonolaurat, Sorbitanmonoisostearat, Sorbitanmonolaurat, Sorbitanmonocaprylat, Sorbitansesquistearat, Sorbitanmonoisooleat, Saccharosedistearat, Cetylalkohol, Stearylalkohol, Arachidylalkohol, Behenylalkohol, Isobehenylalkohol, 2-Ethylhexylglycerinether, Selachylalkohol, Chimylalkohol, Polyethylenglycol(2)stearyl-ether (Steareth-2), Glycerylsorbitanstearat, Polyglyceryl-4-Isostearat, Polyglyceryl-2-sesquiisostearat, PEG-7-hydrogeniertes Ricinusöl, Isostearyldiglycerylsuccinat, PEG-5-Cholesterylether, PEG-30 Dipolyhydroxystearat, Decaglycerylheptaoleat, Polyglyce-ryl-3-diisostearat, PEG-8 Distearat, Diglycerin Dipolyhydroxystearat, Glycerinisostearat, Sorbitanisostearat, Polyglyceryl-3-methylglucosedistearat, PEG-2 Stearat, PEG-45/Dodecylglycolcopolymer, PEG-22/Dodecylglycolcopolymer und Methoxy PEG- 22/Dodecyl Glycol Copolymer.

Die erfindungsgemäßen Zusammensetzungen sind weiterhin dadurch gekennzeichnet, dass mindestens ein Polyethylenglycolether eines linearen oder verzweigten C12 - C22-Alkanols mit 20 - 150 Ethylenoxideinheiten enthalten ist. Diese Verbindungen sind als Öl-in-Wasser-Emulgatoren bekannt. Überraschend wurde festgestellt, dass der Zusatz derartiger Polyethylenglycolether eines linearen oder verzweigten C12 - C22-Alkanols mit 20 - 150 Ethylenoxideinheiten zu einer stark verbesserten Wirkstofffreisetzung des schweißhemmenden Wirkstoffs führt. Besonders überraschend war, dass längst nicht alle Öl-in-Wasser-Emulgatoren diesen begünstigenden Effekt auf die erfindungsgemäßen Zusammensetzungen ausüben, auch nicht solche, die im selben HLB-Wertbereich liegen wie die erfindungsgemäß bevorzugten Polyethylenglycolether eines linearen oder verzweigten C12 - C22-Alkanols mit 20-150 Ethylenoxideinheiten.
Bevorzugte erfindungsgemäße Zusammensetzungen sind dadurch gekennzeichnet, dass der mindestens eine Polyethylenglycolether eines linearen oder verzweigten C12 - C22-Alkanols mit 20 - 150 Ethylenoxideinheiten ausgewählt ist aus Laureth-20, Laureth-25, Laureth-30, Laureth-35, Laureth-40, Laureth-45, Laureth-50, Laureth-55, Laureth-60, Laureth-65, Laureth-70, Laureth-75, Laureth-80, Laureth-85, Laureth-90, Laureth-95, Laureth-100, Myristeth-20, Myristeth-25, Myristeth-30, Myristeth-35, Myristeth-40, Myristeth-45, Myristeth-50, Myristeth-55, Myristeth-60, Myristeth-65, Myristeth-70, Myristeth-75, Myristeth-80, Myristeth-85, Myristeth-90, Myristeth-95, Myristeth-100, Ceteth-20, Ceteth-25, Ceteth-30, Ceteth-35, Ceteth-40, Ceteth-45, Ceteth-50, Ceteth-55, Ceteth-60, Ceteth-65, Ceteth-70, Ceteth-75, Ceteth-80, Ceteth-85, Ceteth-90, Ceteth-95, Ceteth-100, Ceteth-110, Ceteth-120, Ceteth-130, Ceteth-140, Ceteth-150, Isoceteth-20, Isoceteth-25, Isoceteth-30, Isoceteth-35, Isoceteth-40, Isoceteth-45, Isoceteth-50, Isoceteth-55, Isoceteth-60, Isoceteth-65, Isoceteth-70, Isoceteth-75, Isoceteth-80, Isoceteth-85, Isoceteth-90, Isoceteth-95, Isoceteth-100, Isoceteth-110, Isoceteth-120, Isoceteth-130, Isoceteth-140, Isoceteth-150, Steareth-20, Steareth-25, Steareth-30, Steareth-35, Steareth-40, Steareth-45, Steareth-50, Steareth-55, Steareth-60, Steareth-65, Steareth-70, Steareth-75, Steareth-80, Steareth-85, Steareth-90, Steareth-95, Steareth-100, Steareth-110, Steareth-120, Steareth-130, Steareth-140, Steareth-150, Isosteareth-20, Isosteareth-25, Isosteareth-30, Isosteareth-35, Isosteareth-40, Isosteareth-45, Isosteareth-50, Isosteareth-55, Isosteareth-60, Isosteareth-65, Isosteareth-70, Isosteareth-75, Isosteareth-80, Isosteareth-85, Isosteareth-90, Isosteareth-95, Isosteareth-100, Isosteareth-110, Isosteareth-120, Isosteareth-130, Isosteareth-140, Isosteareth-150, Arachideth-20, Arachideth-25, Arachideth-30, Arachideth-35, Arachideth-40, Arachideth-45, Arachideth-50, Arachideth-55, Arachideth-60, Arachideth-65, Arachideth-70, Arachideth-75, Arachideth-80, Arachideth-85, Arachideth-90, Arachideth-95, Arachideth-100, Arachideth-110, Arachideth-120, Arachideth-130, Arachideth-140, Arachideth-150, Beheneth-20, Beheneth-25, Beheneth-30, Beheneth-35, Beheneth-40, Beheneth-45, Beheneth-50, Beheneth-55, Beheneth-60, Beheneth-65, Beheneth-70, Beheneth-75, Beheneth-80, Beheneth-85, Beheneth-90, Beheneth-95, Beheneth-100, Beheneth-110, Beheneth-120, Beheneth-130, Beheneth-140, Beheneth-150, sowie Mischungen hiervon. Außerordentlich bevorzugt ist Steareth-100.
Bevorzugte erfindungsgemäße Zusammensetzungen sind dadurch gekennzeichnet, dass der mindestens eine Polyethylenglycolether eines linearen oder verzweigten C12 - C22-Alkanols mit 20 - 150 Ethylenoxideinheiten in einer Gesamtmenge von 1 - 4 Gew.-%, bevorzugt 1,5 - 3,5, besonders bevorzugt 2 - 3 Gew.-%, außerordentlich bevorzugt 2,2 - 2,6 Gew.%, jeweils bezogen auf das Gesamtgewicht der Emulsion, enthalten ist.

Weitere bevorzugte erfindungsgemäße Zusammensetzungen sind dadurch gekennzeichnet, dass Steareth-100 in einer Gesamtmenge von 1 - 4 Gew.%, bevorzugt 1,5 - 3,5, besonders bevorzugt 2 - 3 Gew.-%, außerordentlich bevorzugt 2,2 - 2,6 Gew.%, jeweils bezogen auf das Gesamtgewicht der Emulsion, enthalten ist.

Weitere bevorzugte erfindungsgemäße Zusammensetzungen sind dadurch gekennzeichnet, dass 1 - 10 Gew.-%, bevorzugt 2-8 Gew.-%, besonders bevorzugt 3-7 Gew.-%, außerordentlich bevorzugt 4 - 6 Gew.-%, Ethanol enthalten sind, jeweils bezogen auf das Gesamtgewicht der Emulsion. Ein solcher Ethanolgehalt unterstützt vorteilhaft die Emulsionsstabilität und die Transparenz.

Die Viskositätsangaben beziehen sich auf Messungen mit einem Rotationsviskosimeter der Firma Brookfield unter Auswahl der Spindel und der Umdrehungszahl, wie sie in dem Handbuch "More Solutions to Sticky Problems" der Firma Brookfield empfohlen ist:
bei Verwendung von T-Spindeln und Helipath:

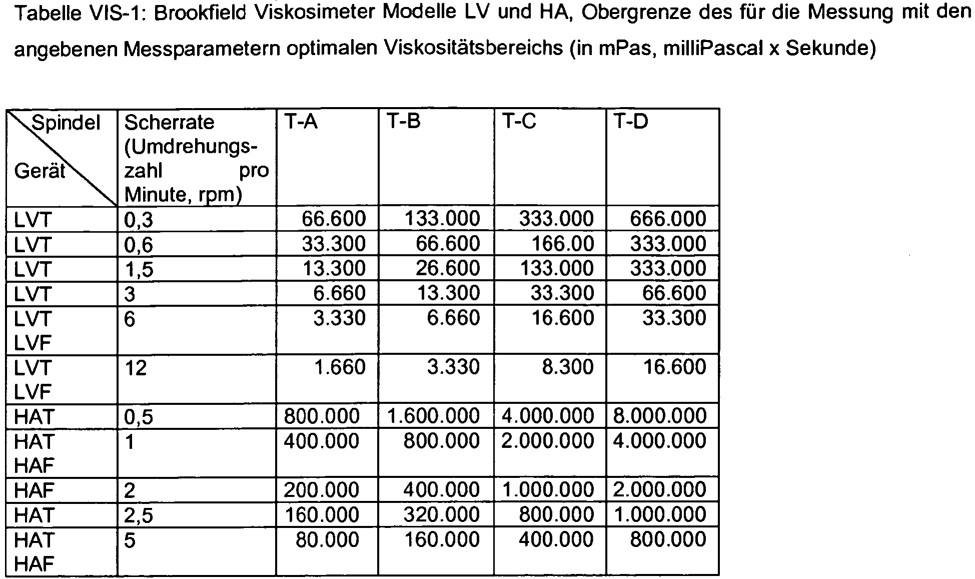

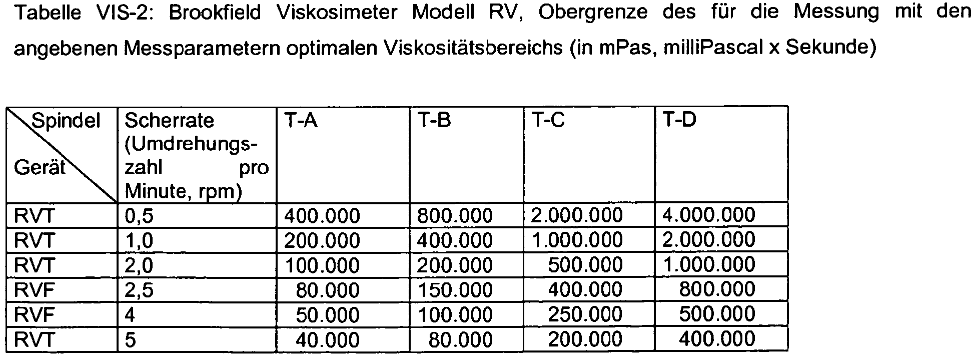

Die angegebene Viskosität stellt den oberen Grenzwert für den optimalen Messbereich für die jeweilige Spindel-Umdrehungszahl-Kombination dar. Falls für einen Viskositätsbereich zwei verschiedene Messparameter-Kombinationen möglich sind, wird die Spindel-Umdrehungszahl-Kombination gewählt, die den höheren Skalenteil-Wert liefert. Weiterhin beziehen sich die Viskositätsangaben auf die Zusammensetzung 24 Stunden nach Herstellung und bei einer Temperatur von 21 °C, gemessen mit einem Helipath.

Erfindungsgemäß bevorzugte Zusammensetzungen mit einer bevorzugten Viskosität sind besonders gut zur Applikation mit einem Kugelapplikator oder aus einem Gelspender geeignet. Um die Transparenz der Zusammensetzung für den Verbraucher deutlich zu machen, ist eine transparente Verpackung bevorzugt.

Weitere erfindungsgemäß bevorzugte Zusammensetzungen können Zusatzstoffe enthalten, wie Deodorantwirkstoffe, bevorzugt 2-Ethylhexylglycerinether (Sensiva SC 50), UV-Filter, Antioxidantien, desensibilisierende Wirkstoffe, wie Aminosäuren, Proteine und Proteinhydrolysate, Vitamine und Vitaminvorstufen, insbesondere Panthenol, Konservierungsmittel und antibakterielle Wirkstoffe.

Die nachstehenden Beispiele sollen die Erfindung verdeutlichen, ohne sie hierauf zu beschränken.

Transparentes schweißhemmendes W/O-Emulsionsgel

| | |
|---|---|
| Diethylhexylcarbonat | 12 Gew.-% |
| Cetyl PEG/PPG-10/1 Dimethicone | 2 Gew.-% |
| PPG-3 Myristylether | 2 Gew.-% |
| Ethanol (96 %) | 5 Gew.-% |
| Parfümöl | 0,6 Gew.-% |
| Aluminium Zirconium Tetrachlorohydrex Gly | 21 Gew.-% |
| 1,2-Propylenglycol | 24,4 Gew.-% |
| Dipropylenglycol | 13,4 Gew.-% |
| Steareth-100 | 2 Gew.-% |
| Wasser | 17,6 Gew.-% |

Aus einem transparenten Gelspender wurde dieses transparente schweißhemmende W/O-Emulsionsgel auf die Haut im Achselbereich aufgetragen. Es wurde eine lang anhaltende schweißhemmende Wirkung beobachtet.

## Patentansprüche

1. Transparente schweißhemmende Zusammensetzung in Form einer Wasser-in-ÖI-Emulsion, enthaltend
a) 10-20 Gew.-% äußere Ölphase, enthaltend
a.i) mindestens einen symmetrischen, unsymmetrischen oder cyclischen Ester der Kohlensäure mit linearen oder verzweigten C6 - C22-Alkanolen,
a.ii) mindestens ein Anlagerungsprodukt von 1 - 14 Propylenoxid-Einheiten an ein- oder mehrwertige C₃₋₁₆-Alkanole sowie
a.iii) 0 bis maximal 3 Gew.-% Cyclomethicone,
b) 75 - 90 Gew.-% dispergierte wässrige Phase mit mindestens einem schweißhemmenden Wirkstoff, ausgewählt aus Aluminium-Zirconium-Verbindungen, und mindestens einer Verbindung, ausgewählt aus wasserlöslichen mehrwertigen C₂ - C₉-Alkanolen mit 2 - 6 Hydroxylgruppen und wasserlöslichen Polyethylenglycolen mit 3-20 Ethylenoxid-Einheiten,
c) mindestens einen Silicon-basierten Wasser-in-Öl-Emulgator mit mindestens einem Alkylsubstituenten R mit 4 - 20 Kohlenstoffatomen,
d) mindestens einen Polyethylenglycolether eines linearen oder verzweigten C12 - C22-Alkanols mit 20-150 Ethylenoxideinheiten,
e) keinen Silicon-freien Wasser-in-Öl-Emulgator,
wobei alle Gew.-%-Angaben auf das Gesamtgewicht der Emulsion bezogen sind.

2. Zusammensetzung gemäß Anspruch 1, **dadurch gekennzeichnet, dass** die Ölkomponenten a.i) und a.ii) in einem Gewichtsverhältnis von 4-19 vorliegen.

3. Zusammensetzung gemäß Anspruch 1 oder 2, **dadurch gekennzeichnet, dass** der mindestens eine Polyethylenglycolether eines linearen oder verzweigten C12 - C22-Alkanols mit 20 - 150 Ethylenoxideinheiten in einer Gesamtmenge von 1 - 4 Gew.-%, bezogen auf das Gesamtgewicht der Emulsion, enthalten ist.

4. Zusammensetzung gemäß einem der Ansprüche 1, 2 oder 3, **dadurch gekennzeichnet, dass** der mindestens eine Polyethylenglycolether eines linearen oder verzweigten C12 - C22-Alkanols mit 20-150 Ethylenoxideinheiten ausgewählt ist aus Laureth-20, Laureth-25, Laureth-30, Laureth-35, Laureth-40, Laureth-45, Laureth-50, Laureth-55, Laureth-60, Laureth-65, Laureth-70, Laureth-75, Laureth-80, Laureth-85, Laureth-90, Laureth-95, Laureth-100, Myristeth-20, Myristeth-25, Myristeth-30, Myristeth-35, Myristeth-40, Myristeth-45, Myristeth-50, Myristeth-55, Myristeth-60, Myristeth-65, Myristeth-70, Myristeth-75, Myristeth-80, Myristeth-85, Myristeth-90, Myristeth-95, Myristeth-100, Ceteth-20, Ceteth-25, Ceteth-30, Ceteth-35, Ceteth-40, Ceteth-45, Ceteth-50, Ceteth-55, Ceteth-60, Ceteth-65, Ceteth-70, Ceteth-75, Ceteth-80, Ceteth-85, Ceteth-90, Ceteth-95, Ceteth-100, Ceteth-110, Ceteth-120, Ceteth-130, Ceteth-140, Ceteth-150, Isoceteth-20, Isoceteth-25, Isoceteth-30, Isoceteth-35, Isoceteth-40, Isoceteth-45, Isoceteth-50, Isoceteth-55, Isoceteth-60, Isoceteth-65, Isoceteth-70, Isoceteth-75, Isoceteth-80, Isoceteth-85, Isoceteth-90, Isoceteth-95, Isoceteth-100, Isoceteth-110, Isoceteth-120, Isoceteth-130, Isoceteth-140, Isoceteth-150, Steareth-20, Steareth-25, Steareth-30, Steareth-35, Steareth-40, Steareth-45, Steareth-50, Steareth-55, Steareth-60, Steareth-65, Steareth-70, Steareth-75, Steareth-80, Steareth-85, Steareth-90, Steareth-95, Steareth-100, Steareth-110, Steareth-120, Steareth-130, Steareth-140, Steareth-150, Isosteareth-20, Isosteareth-25, Isosteareth-30, Isosteareth-35, Isosteareth-40, Isosteareth-45, Isosteareth-50, Isosteareth-55, Isosteareth-60, Isosteareth-65, Isosteareth-70, Isosteareth-75, Isosteareth-80, Isosteareth-85, Isosteareth-90, Isosteareth-95, Isosteareth-100, Isosteareth-110, Isosteareth-120, Isosteareth-130, Isosteareth-140, Isosteareth-150, Arachideth-20, Arachideth-25, Arachideth-30, Arachideth-35, Arachideth-40, Arachideth-45, Arachideth-50, Arachideth-55, Arachideth-60, Arachideth-65, Arachideth-70, Arachideth-75, Arachideth-80, Arachideth-85, Arachideth-90, Arachideth-95, Arachideth-100, Arachideth-110, Arachideth-120, Arachideth-130, Arachideth-140, Arachideth-150, Beheneth-20, Beheneth-25, Beheneth-30, Beheneth-35, Beheneth-40, Beheneth-45, Beheneth-50, Beheneth-55, Beheneth-60, Beheneth-65, Beheneth-70, Beheneth-75, Beheneth-80, Beheneth-85, Beheneth-90, Beheneth-95, Beheneth-100, Beheneth-110, Beheneth-120, Beheneth-130, Beheneth-140, Beheneth-150, sowie Mischungen hiervon.

5. Zusammensetzung gemäß einem der Ansprüche 1 - 4, **dadurch gekennzeichnet, dass** die mindestens eine Verbindung, ausgewählt aus wasserlöslichen mehrwertigen C₂ - C₉-Alkanolen mit 2 - 6 Hydroxylgruppen und wasserlöslichen Polyethylenglycolen mit 3-20 Ethylenoxid-Einheiten, in einer Gesamtmenge von 20 - 60 Gew.-%, bezogen auf das Gesamtgewicht der Emulsion, enthalten sind.

6. Zusammensetzung gemäß einem der Ansprüche 1-5, **dadurch gekennzeichnet, dass** der mindestens eine Silicon-basierte Wasser-in Öl-Emulgator mit mindestens einem Alkylsubstituenten R mit 4 - 20 Kohlenstoffatomen in einer Gesamtmenge von 1-4 Gew.-%, bezogen auf das Gesamtgewicht der Emulsion, enthalten ist.

7. Zusammensetzung gemäß einem der Ansprüche 1-6, **dadurch gekennzeichnet, dass** die schweißhemmenden Wirkstoffe in einer Gesamtmenge von 10 - 22 Gew.-%, bezogen auf den Aktivsubstanzgehalt nach US Pharmacopeia pro Gesamtgewicht der Emulsion, enthalten sind.

8. Zusammensetzung gemäß einem der Ansprüche 1-7, **dadurch gekennzeichnet, dass** 1-10 Gew.-%, bezogen auf das Gesamtgewicht der Emulsion, an Ethanol enthalten sind.

9. Zusammensetzung gemäß einem der Ansprüche 1-8, **gekennzeichnet durch** eine Viskosität von 30.000 bis 150.000 mPas (21 °C).

10. Nicht-therapeutisches, kosmetisches Verfahren zur Reduzierung der Schweißbildung, **dadurch gekennzeichnet, dass** eine Zusammensetzung gemäß einem der Ansprüche 1-9 auf die Haut, bevorzugt auf die Haut im Achselbereich, aufgetragen wird.

## Claims

1. A transparent perspiration-inhibiting composition in the form of a water-in-oil emulsion, containing
a) 10 to 20 wt% external oil phase, containing
a.i) at least one symmetrical, asymmetrical, or cyclic ester of carbonic acid with linear or branched C6 to C22 alkanols,
a.ii) at least one addition product of 1 to 14 propylene oxide units with univalent or polyvalent C₃₋₁₆ alkanols, and
a.iii) 0 to a maximum of 3 wt% cyclomethicone,
b) 75 to 90 wt% dispersed aqueous phase having at least one perspiration-inhibiting active substance selected from aluminum-zirconium compounds, and having at least one compound selected from water-soluble polyvalent C₂ to C₉ alkanols having 2 to 6 hydroxyl groups and water-soluble polyethylene glycols having 3 to 20 ethylene oxide units,
c) at least one silicone-based water-in-oil emulsifier having at least one alkyl substituent R having 4 to 20 carbon atoms,
d) at least one polyethylene glycol ether of a linear or branched C12 to C22 alkanol with 20 to 150 ethylene oxide units,
e) no silicone-free water-in-oil emulsifier,
all the indications of wt% referring to the total weight of the emulsion.

2. The composition according to Claim 1, **characterized in that** the oil components a.i) and a.ii) are present at a weight ratio from 4 to 19.

3. The composition according to Claim 1 or 2, **characterized in that** the at least one polyethylene glycol ether of a linear or branched C12 to C22 alkanol with 20 to 150 ethylene oxide units is contained in a total quantity from 1 to 4 wt%, based on the total weight of the emulsion.

4. The composition according to one of Claims 1, 2, or 3, **characterized in that** the at least one polyethylene glycol ether of a linear or branched C12 to C22 alkanol with 20 to 150 ethylene oxide units is selected from Laureth-20, Laureth-25, Laureth-30, Laureth-35, Laureth-40, Laureth-45, Laureth-50, Laureth-55, Laureth-60, Laureth-65, Laureth-70, Laureth-75, Laureth-80, Laureth-85, Laureth-90, Laureth-95, Laureth-100, Myristeth-20, Myristeth-25, Myristeth-30, Myristeth-35, Myristeth-40, Myristeth-45, Myristeth-50, Myristeth-55, Myristeth-60, Myristeth-65, Myristeth-70, Myristeth-75, Myristeth-80, Myristeth-85, Myristeth-90, Myristeth-95, Myristeth-100, Ceteth-20, Ceteth-25, Ceteth-30, Ceteth-35, Ceteth-40, Ceteth-45, Ceteth-50, Ceteth-55, Ceteth-60, Ceteth-65, Ceteth-70, Ceteth-75, Ceteth-80, Ceteth-85, Ceteth-90, Ceteth-95, Ceteth-100, Ceteth-110, Ceteth-120, Ceteth-130, Ceteth-140, Ceteth-150, Isoceteth-20, Isoceteth-25, Isoceteth-30, Isoceteth-35, Isoceteth-40, Isoceteth-45, Isoceteth-50, Isoceteth-55, Isoceteth-60, Isoceteth-65, Isoceteth-70, Isoceteth-75, Isoceteth-80, lsoceteth-85, lsoceteth-90, Isoceteth-95, Isoceteth-100, Isoceteth-110, Isoceteth-120, Isoceteth-130, Isoceteth-140, Isoceteth-150, Steareth-20, Steareth-25, Steareth-30, Steareth-35, Steareth-40, Steareth-45, Steareth-50, Steareth-55, Steareth-60, Steareth-65, Steareth-70, Steareth-75, Steareth-80, Steareth-85, Steareth-90, Steareth-95, Steareth-100, Steareth-110, Steareth-120, Steareth-130, Steareth-140, Steareth-150, Isosteareth-20, Isosteareth-25, Isosteareth-30, Isosteareth-35, Isosteareth-40, Isosteareth-45, Isosteareth-50, Isosteareth-55, Isosteareth-60, Isosteareth-65, Isosteareth-70, Isosteareth-75, Isosteareth-80, Isosteareth-85, Isosteareth-90, Isosteareth-95, Isosteareth-100, Isosteareth-110, Isosteareth-120, Isosteareth-130, Isosteareth-140, Isosteareth-150, Arachideth-20, Arachideth-25, Arachideth-30, Arachideth-35, Arachideth-40, Arachideth-45, Arachideth-50, Arachideth-55, Arachideth-60, Arachideth-65, Arachideth-70, Arachideth-75, Arachideth-80, Arachideth-85, Arachideth-90, Arachideth-95, Arachideth-100, Arachideth-110, Arachideth-120, Arachideth-130, Arachideth-140, Arachideth-150, Beheneth-20, Beheneth-25, Beheneth-30, Beheneth-35, Beheneth-40, Beheneth-45, Beheneth-50, Beheneth-55, Beheneth-60, Beheneth-65, Beheneth-70, Beheneth-75, Beheneth-80, Beheneth-85, Beheneth-90, Beheneth-95, Beheneth-100, Beheneth-110, Beheneth-120, Beheneth-130, Beheneth-140, Beheneth-150, and mixtures thereof.

5. The composition according to one of Claims 1 to 4, **characterized in that** at least one compound selected from water-soluble polyvalent C₂ to C₉ alkanols having 2 to 6 hydroxyl groups and water-soluble polyethylene glycols having 3 to 20 ethylene oxide units is present in a total quantity from 20 to 60 wt%, based on the total weight of the emulsion.

6. The composition according to one of Claims 1 to 5, **characterized in that** the at least one silicone-based water-in-oil emulsifier having at least one alkyl substituent R having 4 to 20 carbon atoms is contained in a total quantity from 1 to 4 wt%, based on the total weight of the emulsion.

7. The composition according to one of Claims 1 to 6, **characterized in that** the perspiration-inhibiting active substances are contained in a total quantity from 10 to 22 wt%, based on the active-substance content according to the U.S. Pharmacopoeia per total weight of the emulsion.

8. The composition according to one of Claims 1 to 7, **characterized in that** 1 to 10 wt% ethanol, based on the total weight of the emulsion it contains.

9. The composition according to one of Claims 1 to 8, **characterized by** a viscosity from 30,000 to 150,000 mPas (21 °C).

10. A non-therapeutic, cosmetic method for reducing perspiration, **characterized in that** a composition according to one of Claims 1 to 9 is applied onto the skin, preferably onto the skin in the underarm area.

## Revendications

1. Composition anti-transpirante, transparente, sous forme d'une émulsion eau-dans-huile, contenant
a) 10-20% en poids d'une phase huileuse externe, contenant
a.i) au moins un ester symétrique, non symétrique ou cyclique de l'acide carbonique, avec des C₆-C₂₂-alcanols linéaires ou ramifiés,
a.ii) au moins un produit d'addition de 1-14 unités d'oxyde de propylène sur des C₃₋₁₆-alcanols monovalents ou polyvalents, ainsi que
a.iii) 0 à maximum 3% en poids de cyclométhicone,
b) 75-90% en poids de phase aqueuse dispersée contenant au moins une substance active anti-transpirante, choisie parmi les composés d'aluminium-zirconium et au moins un composé, choisi parmi les C₂-C₉-alcanols polyvalents solubles dans l'eau comprenant 2-6 groupes hydroxyle et les polyéthylèneglycols solubles dans l'eau comprenant 3-20 unités d'oxyde d'éthylène,
c) au moins un émulsifiant eau-dans-huile à base de silicone, comprenant au moins un substituant alkyle R comprenant 4-20 atomes de carbone,
d) au moins un polyéthylèneglycoléther d'un C₁₂-C₂₂-alcanol linéaire ou ramifié comprenant 20-150 unités d'oxyde d'éthylène,
e) et ne contenant pas d'émulsifiant eau-dans-huile exempt de silicone,
toutes les indications en % en poids se rapportant au poids total de l'émulsion.

2. Composition selon la revendication 1, **caractérisée en ce que** les composants huileux a.i) et a.ii) se trouvent dans un rapport pondéral de 4-19.

3. Composition selon la revendication 1 ou 2, **caractérisée en ce que** ledit au moins un polyéthylèneglycoléther d'un C₁₂-C₂₂-alcanol linéaire ou ramifié comprenant 20-150 unités d'oxyde d'éthylène est contenu en une quantité totale de 1-4% en poids, par rapport au poids total de l'émulsion.

4. Composition selon l'une quelconque des revendications 1, 2 ou 3, **caractérisée en ce que** ledit au moins un polyéthylèneglycoléther d'un C₁₂-C₂₂-alcanol linéaire ou ramifié comprenant 20-150 unités d'oxyde d'éthylène est choisi parmi Laureth-20, Laureth-25, Laureth-30, Laureth-35, Laureth-40, Laureth-45, Laureth-50, Laureth-55, Laureth-60, Laureth-65, Laureth-70, Laureth-75, Laureth-80, Laureth-85, Laureth-90, Laureth-95, Laureth-100, Myristeth-20, Myristeth-25, Myristeth-30, Myristeth-35, Myristeth-40, Myristeth-45, Myristeth-50, Myristeth-55, Myristeth-60, Myristeth-65, Myristeth-70, Myristeth-75, Myristeth-80, Myristeth-85, Myristeth-90, Myristeth-95, Myristeth-100, Ceteth-20, Ceteth-25, Ceteth-30, Ceteth-35, Ceteth-40, Ceteth-45, Ceteth-50, Ceteth-55, Ceteth-60, Ceteth-65, Ceteth-70, Ceteth-75, Ceteth-80, Ceteth-85, Ceteth-90, Ceteth-95, Ceteth-100, Ceteth-110, Ceteth-120, Ceteth-130, Ceteth-140, Ceteth-150, Isoceteth-20, Isoceteth-25, Isoceteth-30, Isoceteth-35, Isoceteth-40, Isoceteth-45, Isoceteth-50, Isoceteth-55, Isoceteth-60, Isoceteth-65, Isoceteth-70, Isoceteth-75, Isoceteth-80, Isoceteth-85, Isoceteth-90, Isoceteth-95, Isoceteth-100, Isoceteth-110, Isoceteth-120, Isoceteth-130, Isoceteth-140, Isoceteth-150, Steareth-20, Steareth-25, Steareth-30, Steareth-35, Steareth-40, Steareth-45, Steareth-50, Steareth-55, Steareth-60, Steareth-65, Steareth-70, Steareth-75, Steareth-80, Steareth-85, Steareth-90, Steareth-95, Steareth-100, Steareth-110, Steareth-120, Steareth-130, Steareth-140, Steareth-150, Isosteareth-20, Isosteareth-25, Isosteareth-30, Isosteareth-35, Isosteareth-40, Isosteareth-45, Isosteareth-50, Isosteareth-55, Isosteareth-60, Isosteareth-65, Isosteareth-70, Isosteareth-75, Isosteareth-80, Isosteareth-85, Isosteareth-90, Isosteareth-95, Isosteareth-100, Isosteareth-110, Isosteareth-120, Isosteareth-130, Isosteareth-140, Isosteareth-150, Arachideth-20, Arachideth-25, Arachideth-30, Arachideth-35, Arachideth-40, Arachideth-45, Arachideth-50, Arachideth-55, Arachideth-60, Arachideth-65, Arachideth-70, Arachideth-75, Arachideth-80, Arachideth-85, Arachideth-90, Arachideth-95, Arachideth-100, Arachideth-110, Arachideth-120, Arachideth-130, Arachideth-140, Arachideth-150, Beheneth-20, Beheneth-25, Beheneth-30, Beheneth-35, Beheneth-40, Beheneth-45, Beheneth-50, Beheneth-55, Beheneth-60, Beheneth-65, Beheneth-70, Beheneth-75, Beheneth-80, Beheneth-85, Beheneth-90, Beheneth-95, Beheneth-100, Beheneth-110, Beheneth-120, Beheneth-130, Beheneth-140, Beheneth-150, ainsi que leurs mélanges.

5. Composition selon l'une quelconque des revendications 1 à 4, **caractérisée en ce que** ledit au moins un composé, choisi parmi les C₂-C₉-alcanols polyvalents, solubles dans l'eau, comprenant 2-6 groupes hydroxyle et les polyéthylèneglycols solubles dans l'eau, comprenant 3-20 unités d'oxyde d'éthylène est contenu en une quantité totale de 20-60% en poids, par rapport au poids total de l'émulsion.

6. Composition selon l'une quelconque des revendications 1 à 5, **caractérisée en ce que** ledit au moins un émulsifiant eau-dans-huile à base de silicone présentant au moins un substituant alkyle R comprenant 4-20 atomes de carbone est contenu en une quantité totale de 1-4% en poids, par rapport au poids total de l'émulsion.

7. Composition selon l'une quelconque des revendications 1 à 6, **caractérisée en ce que** les substances actives anti-transpirantes sont contenues en une quantité totale de 10-22% en poids, par rapport à la teneur en substance active selon la pharmacopée américaine par rapport au poids total de l'émulsion.

8. Composition selon l'une quelconque des revendications 1 à 7, **caractérisée en ce qu'**elle contient 1-10% en poids, par rapport au poids total de l'émulsion, d'éthanol.

9. Composition selon l'une quelconque des revendications 1 à 8, **caractérisée par** une viscosité de 30 000 à 150 000 mPa.s (21°C).

10. Procédé cosmétique, non thérapeutique, pour la réduction de la formation de transpiration, **caractérisé en ce qu'**une composition selon l'une quelconque des revendications 1-9 est appliquée sur la peau, de préférence sur la peau au niveau des aisselles.
